# EUROPEAN PATENT APPLICATION

(11) **EP 4 201 325 A1**
(43) Date of publication of application: **28.06.2023**
(21) Application number: 22214323.2
(22) Date of filing: 16.12.2022
(51) Int. Cl.: A61B 5/12

(54) **SYSTEM AND METHOD FOR HEARING TESTS**

(30) Priority: 21.12.2021 US 202163292126 P
(71) Applicant: Children's Hearing Foundation, Tapei 114 (TW)
(72) Inventor: Chen, Pei-Hua, 114 Tapei (TW); Chu, Chia-Ying, 114 Tapei (TW); Hung, Yu-Chen, 114 Taipei (TW)
(74) Representative: Kretschmann, Dennis

(57) **Abstract**

The present disclosure provides a system for hearing tests comprising: a data storage device, configured to store a database comprising a plurality of word sets, wherein each of the word set comprises a predetermined number of words respectively and represents a corresponding difficulty level; a user interface device, configured to output a speech of a word to a subject and configured for the subject to correspondingly input a response; and, a server device, configured to communicate data with the data storage device and the user interface device, the server device accessing the data storage device and wherein the server device is further configured to: instruct the user interface device to output a speech of a first word to the subject, the first word being selected from the database; in response to the subject inputting the response, determine whether the response corresponds to the first word; based on the result of the determination, select a second word and instruct the user interface device to output a speech of the second word to the subject; repeat the preceding operations; and when a test termination condition is fulfilled, stop the above steps

## Description

### Technical Field

The present invention relates to a system and a method for hearing tests; in particular, the present invention relates to a system and a method for hearing tests that can acquire accurate test results quickly.

### Prior Art

Hearing tests refers to hearing sensitivity assessments conducted by an audiologist on a subject, often conducted by the audiologist using an audiometer, to determine the subject's sensitivity to different frequencies and volume levels.

In hearing tests, the Speech Discrimination Score (Speech Discrimination Score) refers to an indicator audiologist uses to assess the subject's speech reception and discrimination. To achieve a certain accuracy of the test results, in most of the current speech discrimination tests, a certain number (e.g., at least 25 to 50 questions) of questions are administered on the subject to assess the subject's hearing ability in word discrimination. However, if the subject is a young child, the child may not be able to stay focused for a long time due to the relatively large number of questions, which may affect the test results; or during a hearing tracking process, the subject may easily remember the questions due to the need for multiple tests, which may also affect the test results. In addition, during a hearing loss screening process, the subject needs to maintain a high level of concentration for a long period of time to perform the test. Therefore, for both adults and children, it may be difficult to maintain a high level of concentration for a long period of time during the testing process, resulting in inaccurate test results. On the other hand, in the current medical system, audiologists and testing schedules are very limited. How to obtain accurate results quickly and efficiently should be one of the most important issues in the field of hearing tests.

### Summary of Invention

In view of the above, the objective of present invention is to provide an adaptive system and method for hearing tests that can obtain the results of a subject's word discrimination ability with a relatively small amount of test questions and time without affecting the accuracy of the test and can prevent the test results from being interfered by memory factors at the same time.

A first aspect of the disclosure provides a system for hearing tests comprising: a data storage device, configured to store a database comprising a plurality of word sets, wherein each of the word set comprises a predetermined number of words respectively and represents a corresponding difficulty level; a user interface device, configured to output a speech of a word to a subject and configured for the subject to correspondingly input a response; and, a server device, configured to communicate data with the data storage device and the user interface device, the server device accessing the data storage device and wherein the server device is further configured to: instruct the user interface device to output a speech of a first word to the subject, the first word being selected from the database; in response to the subject inputting the response, determine whether the response corresponds to the first word; based on the result of the determination, select a second word and instruct the user interface device to output a speech of the second word to the subject; repeat the preceding operations; and when a test termination condition is fulfilled, stop the above steps.

In an implementation of the system for hearing tests of the first aspect, the server device may be further configured to assess a test result for output when the test termination condition is fulfilled.

In a further implementation of the system for hearing tests of the first aspect, if the response corresponds to the first word, the second word may be selected from a word set of a next higher difficulty level; or if the response does not correspond to the first word, the second word may be selected from a word set of a next lower difficulty level.

In a further implementation of the system for hearing tests of the first aspect, the test termination condition may comprise one of the following: the words output to the subject have been selected from a word set of a highest difficulty level and the test result is a Pass; the words output to the subject are selected from a word set of a highest difficulty level or a word set of a second highest difficulty level; all words in each of the word sets have been output to the subject and the test result is that a predetermined difficulty level has not been reached; a predetermined number of consecutive output of words selected from a word set of a lowest difficulty level to the subject has been achieved; and all words in the word set of the lowest difficulty level have been output to the subject and the test result is a Fail.

In a further implementation of the system for hearing tests of the first aspect, the test termination condition may further comprise: all the words output to the subject have been selected from a word set of a highest difficulty level and the test result is a Pass.

In a further implementation of the system of hearing tests of the first aspect, at the beginning of the hearing tests, the first word may be selected from a word set of a moderate difficulty.

In a further implementation of the system for hearing tests of the first aspect, the words comprised in the database may be graded and compiled according to the difficulty levels based on the Item Response Theory.

In a further implementation of the system for hearing tests of the first aspect, the words comprised in the data base may be compiled using a compiling procedure.

In a further implementation of the system for hearing tests of the first aspect, the compiling procedure may comprise: administering all words to be stored in the database to a group of adults with normal hearing ability; recording their answered results as Pass/Fail; calculating the difficulty of the words based on the total answered results; grading the difficulties using logit as a word difficulty grading unit, which divides the words into word sets corresponding to different difficulty levels.

In a further implementation of the system for hearing tests of the first aspect, the word can be a single word, or a compound word.

In a further implementation of the system for hearing tests of the first aspect, the database may comprise six word sets representing difficulty levels from Level 1 to Level 6 respectively, or ten word sets representing difficulty levels from Level 1 to Level 10 respectively.

In a further implementation of the system for hearing tests of the first aspect, each of the word sets may comprise 20 to 25 words respectively or 25 to 50 words respectively.

In a further implementation of the system for hearing tests of the first aspect, the user interface device may comprise a text input/output device and/or a speech input/output device.

In a further implementation of the system for hearing tests of the first aspect, the subject can input, via the user interface device, one of the following: a phonetic representation corresponding to the word, a picture corresponding to the word, and a speech corresponding to the word.

In a further implementation of the system for hearing tests of the first aspect, the phonetic representation may comprise a phonetic symbol, such as Zhuyin, or a phonetic alphabet, such as Pinyin.

In a further implementation of the system for hearing tests of the first aspect, the system may further comprise a network device, for communication with an external device.

A second aspect of the disclosure provides a computer-implemented method for hearing tests, comprising: providing a speech of a first word to a subject, wherein the first word is selected from a database comprising a plurality of word sets, wherein each of the word sets comprises a predetermined number of words respectively and represents a corresponding difficulty level; receiving from the subject a response to the speech of the first word; comparing the speech of the first word with the response; based on a comparison result of the speech of the first word with the response, selecting a second word and providing a speech of the second word to the subject; repeating the preceding operations; and when a test termination condition is fulfilled, stopping the above steps.

In an implementation of the computer-implemented method for hearing tests of the second aspect, the method may further comprise: assessing a test result for output when the test termination condition is fulfilled.

In a further implementation of the computer-implemented method for hearing tests of the second aspect, the receiving from the subject a response the speech of the first word may further comprise: the subject providing the response in response to the speech of the first word.

In a further implementation of the computer-implemented method for hearing tests of the second aspect, if the response corresponds to the first word, the second word may be selected from a word set of a next higher difficulty level; or if the response does not correspond to the first word, the second word may be selected from a word set of a next lower difficulty level.

In a further implementation of the computer-implemented method for hearing tests of the second aspect, the test termination condition may comprise one of the following: the words output to the subject have been selected from a word set of a highest difficulty level and the test result is a Pass; the words output to the subject are selected from a word set of a highest difficulty level or a word set of a second highest difficulty level; all words in each of the word sets have been output to the subject and the test result is that a predetermined difficulty level has not been reached; a predetermined number of consecutive output of words selected from a word set of a lowest difficulty level to the subject has been achieved; and all words in the word set of the lowest difficulty level have been output to the subject and the test result is a Fail.

In a further implementation of the computer-implemented method for hearing tests of the second aspect, the test termination condition may further comprise: all the words output to the subject have been selected from a word set of a highest difficulty level and the test result is a Pass.

In a further implementation of the computer-implemented method for hearing tests of the second aspect, at the beginning of the hearing tests, the first word may be selected from a word set of a moderate difficulty.

In a further implementation of the computer-implemented method for hearing tests of the second aspect, the words comprised in the database may be graded and compiled according to the difficulty levels based on the Item Response Theory.

In a further implementation of the computer-implemented method for hearing tests of the second aspect, the computer-implemented method may further comprise a compiling procedure

In a further implementation of the computer-implemented method for hearing tests of the second aspect, the words comprised in the data base may be compiled using the compiling procedure.

In a further implementation of the computer-implemented method for hearing tests of the second aspect, the compiling procedure may comprise: administering all words to be stored in the database to a group of adults with normal hearing ability; recording their answered results as Pass/Fail; calculating the difficulty of the words based on the total answered results; grading the difficulties using logit as a word difficulty grading unit, which divides the words into word sets corresponding to different difficulty levels.

In a further implementation of the computer-implemented method for hearing tests of the second aspect, the word can be a single word, or a compound word.

In a further implementation of the computer-implemented method for hearing tests of the second aspect, the database may comprise six word sets representing difficulty levels from Level 1 to Level 6 respectively, or ten word sets representing difficulty levels from Level 1 to Level 10 respectively.

In a further implementation of the computer-implemented method for hearing tests of the second aspect, each of the word sets may comprise 20 to 25 words respectively or 25 to 50 words respectively.

In a further implementation of the computer-implemented method for hearing tests of the second aspect, the input of the subject may comprise one of the following: a phonetic representation corresponding to the word, a picture corresponding to the word, and a speech corresponding to the word.

In a further implementation of the computer-implemented method for hearing tests of the second aspect, the phonetic representation may comprise a phonetic symbol, such as Zhuyin, or a phonetic alphabet, such as Pinyin.

A third aspect of the disclosure provides a computer program comprising instructions which, when the program is executed by a computer, cause the computer to carry out the steps of the method for hearing tests of the second aspect.

A fourth aspect of the disclosure provides a computer-readable storage medium comprising instructions which, when executed by a computer, cause the computer to carry out the steps of the method for hearing tests of the second aspect.

According to a fifth aspect of the present invention, the proposed system for hearing tests comprises: a data storage device, storing a database comprising a plurality of word sets, each of the word sets comprising a predetermined number of words respectively and representing a corresponding difficulty level; a user interface device, for outputting a speech of a word to a subject and for the subject to correspondingly input a response; and a server device, communicating data with the data storage device and the user interface device. According to the idea of the present invention, the server device accesses the data storage device and is configured to: instruct the user interface device to output a speech of a first word to the subject, the first word being selected from the database; in response to the subject inputting the response, determine whether the response corresponds to the first word; based on the result of the determination, select a second word and instruct the user interface device to output a speech of the second word to the subject; repeat the preceding operations; and when a test termination condition is fulfilled, stop the above steps and assess a test result for output.

Based on the above aspect, wherein if the response of the subject corresponds to the first word, the second word is selected from a word set of a next higher difficulty level; or if the response does not correspond to the first word, the second word is selected from a word set of a next lower difficulty level.

Based on the above aspect, wherein the test termination condition comprises one of the following: all the words output to the subject have been selected from a word set of a highest difficulty level and the test result is a Pass; the words output to the subject are selected from a word set of a highest difficulty level or a word set of a second highest difficulty level; all words in each of the word set have been output to the subject and the test result is that a predetermined difficulty level has not been reached; a predetermined number of consecutive output of words selected from a word set of a lowest difficulty level to the subject has been achieved; and all words in the word set of the lowest difficulty level have been output to the subject and the test result is a Fail.

Based on the above aspect, the system for hearing tests of the present invention further comprises a network device for communication with an external device.

Based on the above aspect, the database comprises six sets of words representing difficulty levels from Level 1 to Level 6 respectively.

Based on the above aspect, the database comprises ten sets of words representing difficulty levels from Level 1 to Level 6 respectively.

Based on the above aspect, each of the word sets comprises 20 to 25 words respectively. Based on an alternative idea, each of the word sets comprises 26 to 50 words respectively.

Based on the above aspect, the word can be a single word or a compound word.

Based on the above aspect, wherein the user interface device comprises a text input/output device or a speech input/output device.

Based on the above aspect, wherein the subject can input, via the user interface device, one of the following: a phonetic symbol (Zhuyin) corresponding to the word, a picture corresponding to the word, a phonetic alphabet (Pinyin) corresponding to the word, and a speech corresponding to the word.

According to a sixth aspect of the present invention, the proposed method for hearing tests comprises the following steps: providing a speech of a first word to a subject, wherein the first word is selected from a database comprising a plurality of word sets, wherein each of the word sets comprises a predetermined number of words respectively and represents a corresponding difficulty level; the subject providing a response in response to the speech of the first word; comparing the speech of the first word with the response; based on a comparison result of the speech of the first word with the response, selecting a second word and providing a speech of the second word to the subject, wherein, if the response corresponds to the first word, the second word is selected from a word set of a next higher difficulty level; or if the response does not correspond to the first word, the second word is selected from a word set of a next lower difficulty level; repeating the preceding operations; and when a test termination condition is fulfilled, stopping the above steps and assessing a test result for output.

The preceding aspects and other aspects of the present invention will be better understood in accordance with the following detailed description of non-limiting embodiments and by reference to the accompanying drawings.

### Brief Description of the Drawings

For a better understanding of the features and technical content of the present invention, please refer to the following detailed description of the embodiments of the invention and the accompanying drawings. However, the detailed description and the accompanying drawings are provided for reference and illustration purposes only and are not intended to limit the present invention; wherein.
FIG. 1 is a flow chart illustrating the steps for implementing a method for hearing tests according to the present invention.
FIG. 2 is a block diagram illustrating an embodiment of a system for hearing tests 100 according to the present invention; and
FIG. 3 illustrates an implementation of the method for hearing tests of the present invention.

### Embodiments

The following is an illustration of the concept of the invention by reference to the drawings and using exemplary embodiments. In the drawing and the illustration, similar or identical parts are shown using the same component symbols; furthermore, the drawings are drawn for ease of understanding. The size and shape of the components in the drawings do not correspond to the actual measurements, nor proportional to it. In particular, it should be noted that components not shown in the drawings or described in the specification may be in a form known to persons skilled in the art of the invention.

In the following description, certain specific details (e.g., examples of programming, software modules, user selections, network disruptions, database queries, database structures, hardware modules, hardware circuits, hardware chips, etc.) are provided to provide a thorough understanding of the disclosed embodiments. However, a person skilled in the art of the invention will appreciate that embodiments of the invention may be carried out without one or more of the particular details or with the aid of other methods, components, materials, etc. In other embodiments, the structures, materials or operations known to the public are not shown or described in detail in order to avoid confusion to the form of the invention.

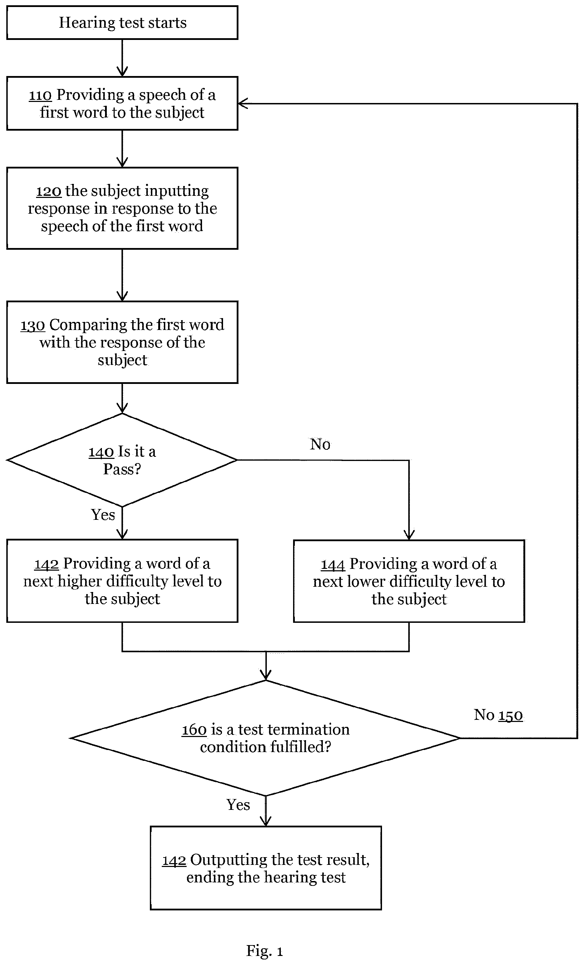

Refer to FIG. 1 for a schematic illustration of the steps for implementing a method for hearing tests 100 according to the present invention. The method for hearing test 100 of the present invention is carried out by a tester (for example, but not limited to an audiologist, hearing aid manufacturer, etc.) when performing a hearing test on a subject using the system for hearing tests of the present invention (described further below), the detailed process as follows:

The method for hearing tests 100 of the present invention starts when the subject wants to perform the hearing test with or without a hearing aid. At step 110, the system for hearing tests of the present invention is used to provide a speech of a first word to the subject, wherein the first word is selected from a database comprising a plurality of word sets, wherein each of the word sets comprises a predetermined number of words respectively and represents a corresponding difficulty level. According to an embodiment of the present invention, the database may comprise, for example, but not limited to, six word sets, or ten word sets, each word set comprising, for example, but not limited to, 20 to 25 words, or 26 to 50 words. According to an embodiment of the present invention, each word set represents a corresponding difficulty level, for example, but not limited to, Level 1 (Level 1) to Level 6 (Level 6), or Level 1 (Level 1) to Level 10 (Level 10), which are gradings compiled based on "Item Response Theory (Item Response Theory, IRT)". For example, in this embodiment, the words for performing the test are divided into six word sets with different difficulty levels, while those skilled in the art should understand that different numbers of gradings can be compiled based on the actual needs of the test, and the number of words in a word set of each grading can be adjusted according to the actual needs of the test.

At step 120, the subject provides a response in response to the speech of the first word. For example, when the first word provided to the subject is "tree", the subject provides a response upon hearing the speech of "tree"; i.e., input his or her response to the system for hearing tests of the present invention.

At step 130, the system compares the speech of the first word provided to the subject with the subject's response, and at step 140, the system selects a second word and provides the speech of the second word to the subject at step 142 or 144 based on whether a comparison result of the speech of the first word and the response is a Pass or a Fail.

In detail, in an embodiment of the present invention, the first word provided by the system to the subject at step 110 is "tree", and the word "tree" belongs to a word set of difficulty Level 2 (Level 2). At step 120, the subject hears the speech of "tree" provided by the system and inputs his or her response to the system, e.g., inputs " 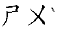 (shu)" using the user interface device of the system (described further below). Then, at step 130, the system determines "trees" as being corresponding to " 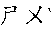 (shu)" and determines based on this that the subject's response is correct or is a Pass (Pass) (step 140), and selects a second word (e.g., "umbrella") from the word set of difficulty Level 3 at step 142, and provides the second word to the subject for test.

On the other hand, if at step 120, after the subject hears the speech of "tree" provided by the system, he or she inputs " 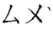 (su)" as response to the system, the system determines "tree" as being not corresponding to " 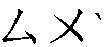 (su)" at step 130, i.e., it determines that the subject's response is incorrect or is a Fail (Fail) (step 140), and select a second word (e.g., "paper") from the word set of difficulty Level 1 at step 144, and provides the second word to the subject for test.

Then, steps 110 to 140 are repeated (step 150) until the system determines that a test termination condition is fulfilled (step 160); finally, the system may assess a test result for output (step 160).

According to an embodiment of the present invention, when the test proceeds to one of the following conditions, it can be determined that a test termination condition is fulfilled, and the hearing test is then terminated:
(A) when all the words output to the subject have been selected from a word set of a highest difficulty level (e.g., Level 6), while the test result assessed by the system is a Pass.
(B) when the words output to the subject are selected from a word set of a highest difficulty level (e.g., Level 6) or a word set of a second highest difficulty level (e.g., Level 5), while the test result assessed by the system is a Pass.
(C) when all words in each of the word sets have been output to the subject and the test result is that a predetermined difficulty level (e.g., Level 5) has not been reached.
(D) when a predetermined number of consecutive output (e.g., 3 times) of words selected from a word set of a lowest difficulty level (e.g., Level 1) to the subject has been achieved; and
(E) when all words in the word set of the lowest difficulty level (e.g., Level 1) have been output to the subject and the subject's response is a Fail.

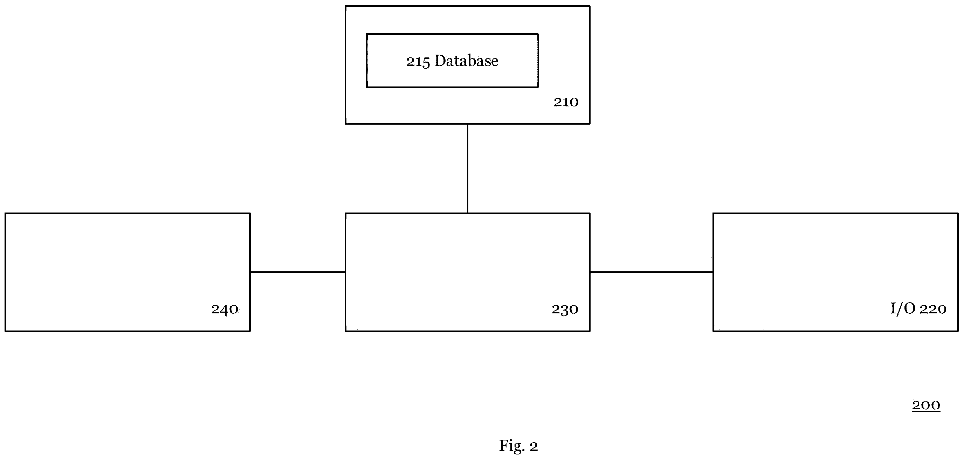

Refer to FIG. 2, which illustrates an embodiment of a system for hearing tests 200 according to the present invention. The system for hearing test 200 of the present invention is used to perform the method for hearing tests of the present invention, and may comprise a data storage device 210, a user interface device 120, and a server device 130 that are connected to each other for data transfer. Alternatively, the system for hearing tests 200 of the present invention may further comprise a network device 240 for the system for hearing tests 200 to communicate with an external device (not shown) for data transfer.

According to an embodiment of the present invention, the data storage device 210 stores a database 215 comprising a plurality of word sets. According to an embodiment of the present invention, each of the word sets comprises a predetermined number of words (for example, but not limited to: 20 to 25 words) respectively and represents a corresponding difficulty level (for example, but not limited to: Level 1 to Level 6 (Level1 ~Level6)). The user interface device 220 is provided for outputting a speech of a word to a subject and for the subject to correspondingly input a response. The server device 230 communicates data with the data storage device 210 and the user interface device 220, and accesses the data storage device 210, receives subject's input from the user interface device 220, and instructs the user interface device 220 to provide speech output to the subject. According to an embodiment of the present invention, the server device 230 is configured to perform the following operations to perform hearing tests on the subject: instructing the user interface device 220 to output a speech of a first word to the subject, the first word being selected from the database 215; in response to the subject inputting the response, determining whether the response of the subject corresponds to the first word; based on the determination, selecting a second word and instructing the user interface device 220 to output the speech of the second word to the subject, wherein: if the response corresponds to the first word, the second word is selected from a word set of the next higher difficulty level; or if the response does not correspond to the first word, the second word is selected from a word set of the next lower difficulty level. The server device 230 repeats the preceding operations until a test termination condition is fulfilled, stops the above steps, and assess a test result for output. Details of the operations are described above and will not be repeated here.

The system for hearing tests 200 of the present invention may be realized as a computer device or a portable device (e.g., a tablet device, a notebook computer, etc.), and wherein the data storage device 210 may be an internal storage device within the computer device or the portable device, or a separate storage device located outside the computer device or the portable device or a remote separate storage device which is connected by a network communication. The user interface device 220 is provided for user control and may comprise, for example, computer peripherals such as monitors, touch screens, computer keyboards, mouses, and the like. Server device 230 may comprise, for example, several separate components, including, but not limited to: one or more processors/microprocessors, memories (e.g., volatile memories, such as RAMs) to load executable instructions, wherein said executable instructions define the functionality (i.e., the method steps of the present invention) to be performed by the server device 230 under the control of a processor. Alternatively, the user interface device 220 may be an external device independent from the server device 230 (e.g., a tablet device with an independent function), or may be a peripheral device of the server device 230 as described above.

According to an embodiment of the present invention, a word comprised in the database 215 may be a single word, or a compound word; in particular, a word comprised in the database 215 may be a Chinese single word, or a Chinese compound word, and the comprised words comprised therein are graded and compiled according to the difficulty levels based on the Item Response Theory (IRT). In addition, according to an embodiment of the present invention, the subject may enter his or her response via a text input device (e.g., a computer keyboard, a touch screen), a speech input device (e.g., microphone), or other device (e.g., mouse click); in other words, the subject may input via the user interface device 220 one of the following as his or her response: a phonetic symbol (Zhuyin) corresponding to the word, a picture corresponding to the word, a phonetic alphabet (Pinyin) corresponding to the word, and a speech corresponding to the word. For example, when a subject hears the speech of the word "tree" provided to him by the system for hearing tests 200 of the present invention (assuming it belongs to a word set of a Level 2 difficulty as described above), he or she can input his or her response, for example " 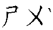 (shu)", via a text input device such as a computer keyboard or a touch screen, or he can input the speech of "tree" via a speech input device such as a microphone, or he can click on the picture or photo representing "tree" in a system screen with a mouse, for the system to compare.

As described above, according to the present invention, the server device 230 is configured to compare whether the response input by the subject corresponds to the word it provides to the subject, and to select a word of the next higher difficulty level (Level 3), or a word of the next lower difficulty level (Level 1), respectively, based on whether the comparison result is a Pass or a Fail. As described above, the system repeatedly performs the test on the subject and ends the test when one of the aforementioned test termination conditions is fulfilled.

As described above, according to an embodiment of the present invention, all words comprised in the database are classified and compiled based on IRT into six word sets representing different difficulty gradings (only as an example), and each word set comprises a certain number of words (for example, but not limited to 20 to 25). For example, according to an embodiment of the invention, Level 6 (Level 6) represents the most difficult word set and Level 1 (Level 1) represents the least difficult word set. The compiling procedure comprises: administering all words to a group of adults with normal hearing ability, and recording their answered results as Pass/Fail (Pass/Fail); calculating the difficulty of the words based on the total answered results; grading the difficulties using logit (logit) as a word difficulty grading unit, which divides the words into six Levels (Level). While performing hearing tests, a subject is tested by using each level as unit. The cut-off points of the six difficulty levels (Levels 1 to 6) described above are shown in the table below.

| Gradings | Level 1 | Level 2 | Grade 3 | Level 4 | Grade 5 | Grade 6 |
|---|---|---|---|---|---|---|
| Number of words | 4 | 11 | 11 | 11 | 8 | 5 |
| Average (logits) | -3.33 | -1.57 | -0.37 | 0.38 | 1.35 | 2.35 |
| Range (logits) | -3.95~-2.70 | -1.93~-1.05 | -0.80∼-0.12 | 0.24~0.73 | 1.05~1.88 | 2.08~2.53 |

For example, in the present invention, the Mandarin Monosyllable Recognition Test (Mandarin Monosyllable Recognition Test, MMRT) is used as the experimental material, and all the words questions in the MMRT are graded for the difficulty as outlined above based on IRT, and tested by the adaptive (adaptive) system and method for tests of the present invention. In the following, compared with MMRT, the method for hearing tests of the present invention is called "Adaptive Mandarin Monosyllable Recognition Test (Adaptive Mandarin Monosyllable Recognition Test, AMMRT)".

In the following, statistics are conducted on the test results of the known MMRT and the AMMRT performed on 37 adults to test the consistency of the two tests. Taking the test results of the subjects under conventional test procedure as base line (i.e. MMRT), while comparing the consistency of the test results under the adaptive method for hearing tests of the present invention (AMMRT), statistical methods are performed in the form of chi-square test (chi-square test). The chi-square test is mainly used for testing the consistency of two results, which is here comparing the consistency of the test results of the two methods for hearing tests; when the p-value of the analysis result is less than .05, it means that the statistical significance level is reached, which means that the consistency test is passed.

According to the results of the analysis, the p-value of the chi-square test was .014, indicating a statistically significant level (χ²= 6.03, p = .014), which means here that the results of MMRT and AMMRT have achieved a significant consistency. In other words, the subjects who can pass the known MMRT will also pass the AMMRT; this means that the adaptive system and method for hearing tests of the present invention can replace the time-consuming known MMRT test method as an efficient and accurate method for hearing tests. The detailed statistics are shown in the following table.

### chi-square test (n = 37)

| | AMMRT | | χ² | df | p |
|---|---|---|---|---|---|
| MMRT | Fail | Pass | | | |
| Fail | 3 | 10 | 6.03 | 1 | .014 |
| Pass | 0 | 24 | | | |

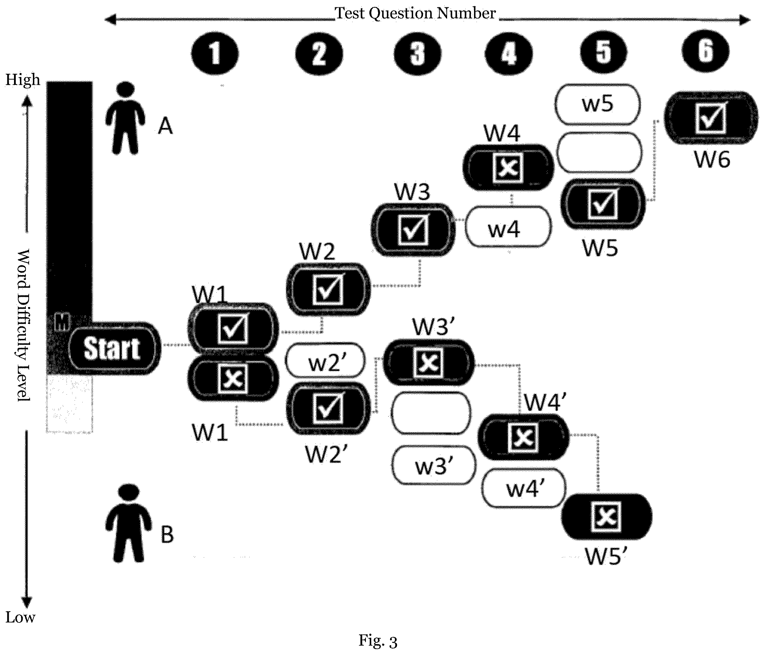

Refer to FIG. 3, which illustrates the implementation of the adaptive hearing test of the present invention, i.e., the process of performing the hearing test to subject A and subject B using the system for hearing tests of the present invention. In this embodiment, the difficulty level of the hearing test is divided into Levels 1 to 6, but the invention is not limited to this. As shown in Figure 3, at the beginning of the hearing test, both subject A and subject B are provided with a first word W1 belonging to a same word set of a moderate difficulty, where subject A's response corresponds to the first word W1 (correct response) and the result is displayed as a "Pass" (indicated by "V"). Based on the correct response of subject A, the system then selects and provides a second word W2 to subject A that is more difficult than the first word W1. The the test result also shows that subject A's response is correct (Pass); subject A then also passes the third word W3 that is more difficult than the second word W2, but fails to correctly respond to the fourth word W4 that is more difficult than the third word W3 (the test result is a Fail, indicated by "X"); then, based on this result, the system selects the word W5 that is slightly less difficult than the word W4 for performing the test and continues to increase the difficulty of the word after subject A passes. Finally, subject A responds correctly to the most difficult word W6, fulfilling the aforementioned test termination condition (A), and then the test is terminated and the test result of passing the hearing test is obtained.

On the other hand, the response of subject B during the test does not correspond to the word W1, i.e., a Fail ("X"); the system then selected a word W2', which is less difficult than the word W1, to perform a second test on subject B. Although subject B passes the second test which triggers the system to select the word W3', which is more difficult than the word W2', to perform the test, the subsequent test results show that subject B does not pass the test words W3', W4', and W5', with decreasing difficulties. Since subject B fails three consecutive tests and the aforementioned test termination condition (D) was fulfilled, the test is terminated and the test result shows that subject B fails the hearing test.

From the above, it can be learned that in the fastest case, the hearing test result of A can be obtained by performing tests of six questions on subject A, while subject B confirms that he or she has failed the hearing test after receiving tests of five questions. Even considering the case where the responses of the subjects to words of same difficulty levels are recurring "pass" or "fail" (for example, the number of questions for w2', w3', w4, w4', w5, etc. shown in Figure 3), causing the number of the words for performing the test to increase slightly, while comparing to known test method where at least 25 to 50 questions need to be performed to calculate the percentage of correctness, the adaptive system and method for hearing test of the present invention can indeed significantly reduce the amount of test questions and the time required to perform the test, and also obtains similar accurate hearing test results (i.e., the test results are highly consistent with the known test method).

As described above, the system and method for hearing tests of the present invention is an adaptive system and method for hearing tests constructed based on IRT. By integrating a certain number of words with similar properties as groups of test questions, it can be avoided that the difficulty of the words varies with the ability of the subject, and tests can be performed by selecting words of required difficulty from different groups of test questions. Therefore, the difficulty gap between test questions/words can be reduced. In addition, the subject must at least pass the Level 1 (with the lowest difficulty) test to continue the following test steps, which reduces the frustration and efforts due to the subject's lack of ability when the subject still has to finish the hearing test of all words in known test methods. This is indeed an efficient and accurate system and method for hearing test.

It should be noted that the foregoing embodiments of the present invention are intended only to illustrate the invention and not to limit the scope of the invention. Any modification or variation of the invention by a person skilled in the art shall not depart from the spirit and scope of the invention. The same or similar components in different embodiments, or components denoted by the same component symbols in different embodiments, have the same physical or chemical properties. Further, where appropriate, the aforementioned embodiments of the present invention may be combined or substituted with each other and are not limited to the particular embodiments described above. The connection of a particular component with other components described in one embodiment may also be applied to other embodiments, all of which fall within the scope of the present invention as the accompanying patent application.

### Description of Reference Signs

- 100: method for hearing tests
- 110 to 160: Steps
- 200: system for hearing tests
- 210: Data Storage Device
- 215: Database
- 220: User Interface Device
- 230: Server device
- 240: Network Devices

## Claims

1. A system for hearing tests (200) comprising:
a data storage device (210), configured to store a database (215) comprising a plurality of word sets, wherein each of the word set comprises a predetermined number of words respectively and represents a corresponding difficulty level;
a user interface device (220), configured to output a speech of a word to a subject and
configured for the subject to correspondingly input a response; and,
a server device (230), configured to communicate data with the data storage device (210) and the user interface device (220), the server device (230) accessing the data storage device (210) and wherein the server device (230) is further configured to:
instruct the user interface device (220) to output a speech of a first word to the subject, the first word being selected from the database;
in response to the subject inputting the response, determine whether the response corresponds to the first word;
based on the result of the determination, select a second word and instruct the user interface device to output a speech of the second word to the subject;
repeat the preceding operations; and
when a test termination condition is fulfilled, stop the above steps.

2. The system for hearing tests (200) of claim 1, wherein:
if the response corresponds to the first word (140), the second word is selected from a word set of a next higher difficulty level (142); or
if the response does not correspond to the first word (140), the second word is selected from a word set of a next lower difficulty level (144).

3. The system for hearing tests (200) of claim 1 or 2, wherein the test termination condition comprises one of the following:
the words output to the subject have been selected from a word set of a highest difficulty level and the test result is a Pass;
the words output to the subject are selected from a word set of a highest difficulty level or a word set of a second highest difficulty level;
all words in each of the word sets have been output to the subject and the test result is that a predetermined difficulty level has not been reached;
a predetermined number of consecutive output of words selected from a word set of a lowest difficulty level to the subject has been achieved; and
all words in the word set of the lowest difficulty level have been output to the subject and the test result is a Fail.

4. The system for hearing tests (200) of one of the preceding claims, wherein at the beginning of the hearing tests, the first word is selected from a word set of a moderate difficulty.

5. The system for hearing tests (200) of one of the preceding claims, wherein the words comprised in the database are graded and compiled according to the difficulty levels based on the Item Response Theory.

6. The system for hearing tests (200) of claim 5, wherein the compiling procedure comprises:
administering all words to be stored in the database (215) to a group of adults with normal hearing ability;
recording their answered results as Pass/Fail;
calculating the difficulty of the words based on the total answered results;
grading the difficulties using logit as a word difficulty grading unit, which divides the words into word sets corresponding to different difficulty levels.

7. The system for hearing tests (200) of one of the preceding claims, wherein the word is a single word, or a compound word.

8. The system for hearing tests (200) of one of the preceding claims, wherein the database (215) comprises six word sets representing difficulty levels from Level 1 to Level 6 respectively, or ten word sets representing difficulty levels from Level 1 to Level 10 respectively.

9. The system for hearing tests (200) of one of the preceding claims, wherein each of the word sets comprises 20 to 25 words respectively or 25 to 50 words respectively.

10. The system for hearing tests (200) of one of the preceding claims, wherein the user interface device (220) comprises a text input/output device and/or a speech input/output device.

11. The system for hearing tests (200) of one of the preceding claims, wherein the subject inputs, via the user interface device (220), one of the following: a phonetic representation corresponding to the word, a picture corresponding to the word, and a speech corresponding to the word.

12. The system for hearing tests (200) of one of the proceeding claims, further comprising a network device (240), for communication with an external device.

13. A computer-implemented method (100) for hearing tests, comprising:
providing (110) a speech of a first word to a subject, wherein the first word is selected from a database (215) comprising a plurality of word sets, wherein each of the word sets comprises a predetermined number of words respectively and represents a corresponding difficulty level;
receiving from the subject (120) a response to the speech of the first word;
comparing (130) the speech of the first word with the response;
based on a comparison result of the speech of the first word with the response (140), selecting (142, 144) a second word and providing a speech of the second word to the subject;
repeating (150) the preceding operations; and
when a test termination condition is fulfilled (160), stopping the above steps.

14. A computer program comprising instructions which, when the program is executed by a computer, cause the computer to carry out the steps of the method for hearing tests (100) of claim 13.

15. A computer-readable storage medium comprising instructions which, when executed by a computer, cause the computer to carry out the steps of the method for hearing tests (100) of claim 13.
